# EUROPEAN PATENT APPLICATION

(11) **EP 3 756 679 A1**
(43) Date of publication of application: **30.12.2020**
(21) Application number: 19183317.7
(22) Date of filing: 28.06.2019
(51) Int. Cl.: A61K 38/17, A61P 3/10, A61P 5/50

(54) **COMPOSITIONS FOR USE IN THE TREATMENT OF INSULIN DEFICIENCY CONDITIONS**

(71) Applicant: Université de Genève, 1211 Genève (CH)
(72) Inventor: COPPARI, Roberto, 1206 Genève (CH); RAMADORI, Giorgio, 3014 Bern (CH); MIKROPOULOU, Despoina, 01280 Prevessin-Möens (FR)
(74) Representative: KATZAROV S.A.

(57) **Abstract**

The present disclosure provides compositions and methods for their use in the treatment of insulin deficiency (ID) condition, or an associated symptom, in a subject in need thereof, the compositions comprising a S100 calcium-binding protein A9 (S100A9), a variant or a fragment thereof and insulin, a variant or a fragment thereof.

SEQUENCE LISTING

<110>  Université de Genève

<120>  Compositions for use in the treatment of insulin deficiency
       conditions

<130>  51871/EP

<160>  48

<170>  PatentIn version 3.5

<210>  1
<211>  114
<212>  PRT
<213>  Homo sapiens

<400>  1

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Pro

<210>  2
<211>  91
<212>  PRT
<213>  Unknown

<220>
<223>  Artifical Sequence

<400>  2

Met Thr Cys Lys Met Ser Gln Leu Glu Arg Asn Ile Glu Thr Ile Ile

1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His
                85                  90

<210>  3
<211>  91
<212>  PRT
<213>  unknown

<220>
<223>  Artificial Sequence

<400>  3

Met Lys Leu Gly His Pro Asp Thr Leu Asn Gln Gly Glu Phe Lys Glu
1               5                   10                  15

Leu Val Arg Lys Asp Leu Gln Asn Phe Leu Lys Lys Glu Asn Lys Asn
            20                  25                  30

Glu Lys Val Ile Glu His Ile Met Glu Asp Leu Asp Thr Asn Ala Asp
        35                  40                  45

Lys Gln Leu Ser Phe Glu Glu Phe Ile Met Leu Met Ala Arg Leu Thr
    50                  55                  60

Trp Ala Ser His Glu Lys Met His Glu Gly Asp Glu Gly Pro Gly His
65                  70                  75                  80

His His Lys Pro Gly Leu Gly Glu Gly Thr Pro
                85                  90

<210>  4
<211>  76
<212>  PRT
<213>  unknown

<220>

<223>  Artificial Sequence

<400>  4

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe
65                  70                  75

<210>  5
<211>  76
<212>  PRT
<213>  unknown

<220>
<223>  Artificial Sequence

<400>  5

Met Leu Val Arg Lys Asp Leu Gln Asn Phe Leu Lys Lys Glu Asn Lys
1               5                   10                  15

Asn Glu Lys Val Ile Glu His Ile Met Glu Asp Leu Asp Thr Asn Ala
            20                  25                  30

Asp Lys Gln Leu Ser Phe Glu Glu Phe Ile Met Leu Met Ala Arg Leu
        35                  40                  45

Thr Trp Ala Ser His Glu Lys Met His Glu Gly Asp Glu Gly Pro Gly
    50                  55                  60

His His His Lys Pro Gly Leu Gly Glu Gly Thr Pro
65                  70                  75

<210>  6
<211>  114
<212>  PRT
<213>  unknown

<220>
<223>  Artificial Sequence

<400>  6

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Ala Ala Asp Lys Gln Leu Ser Phe Glu Ala Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Pro

<210>  7
<211>  21
<212>  PRT
<213>  Homo sapiens

<400>  7

Gly Ile Val Glu Gln Cys Cys Thr Ser Ile Cys Ser Leu Tyr Gln Leu
1               5                   10                  15

Glu Asn Tyr Cys Asn
            20

<210>  8
<211>  30
<212>  PRT
<213>  Homo sapiens

<400>  8

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr
            20                  25                  30

<210>  9
<211>  21
<212>  PRT
<213>  unknown

<220>
<223>  Artificial Sequence

<400>  9

Gly Ile Val Glu Gln Cys Cys Thr Ser Ile Cys Ser Leu Tyr Gln Leu
1               5                   10                  15


Glu Asn Tyr Cys Asn
            20


<210>  10
<211>  30
<212>  PRT
<213>  unknown

<220>
<223>  Artificial Sequence

<400>  10

Phe Val Asn Gln His Leu Cys Gly Ser His Leu Val Glu Ala Leu Tyr
1               5                   10                  15


Leu Val Cys Gly Glu Arg Gly Phe Phe Tyr Thr Lys Pro Thr
            20                  25                  30


<210>  11
<211>  21
<212>  PRT
<213>  unknown

<220>
<223>  Artificial Sequence

<400>  11

Gly Ile Val Glu Gln Cys Cys Thr Ser Ile Cys Ser Leu Tyr Gln Leu
1               5                   10                  15


Glu Asn Tyr Cys Asn
            20


<210>  12
<211>  30
<212>  PRT
<213>  unknown

<220>
<223>  Artificial Sequence

<400>  12

Phe Val Lys Gln His Leu Cys Gly Ser His Leu Val Glu Ala Leu Tyr
1               5                   10                  15

Leu Val Cys Gly Glu Arg Gly Phe Phe Tyr Thr Pro Glu Thr
            20                  25                  30

<210>  13
<211>  21
<212>  PRT
<213>  unknown

<220>
<223>  Artificial Sequence

<400>  13

Gly Ile Val Glu Gln Cys Cys Thr Ser Ile Cys Ser Leu Tyr Gln Leu
1               5                   10                  15

Glu Asn Tyr Cys Asn
            20

<210>  14
<211>  30
<212>  PRT
<213>  unknown

<220>
<223>  Artificial Sequence

<400>  14

Phe Val Asn Gln His Leu Cys Gly Ser His Leu Val Glu Ala Leu Tyr
1               5                   10                  15

Leu Val Cys Gly Glu Arg Gly Phe Phe Tyr Thr Asp Lys Thr
            20                  25                  30

<210>  15
<211>  21
<212>  PRT
<213>  unknown

<220>
<223>  Artificial Sequence

<400>  15

Gly Ile Val Glu Gln Cys Cys Thr Ser Ile Cys Ser Leu Tyr Gln Leu
1               5                   10                  15

Glu Asn Tyr Cys Gly
            20

<210>  16
<211>  32
<212>  PRT
<213>  unknown

<220>
<223>  Artificial Sequence

<400>  16

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg Arg
            20                  25                  30

<210>  17
<211>  21
<212>  PRT
<213>  Unknown

<220>
<223>  Artificial Sequence

<400>  17

Gly Ile Val Glu Gln Cys Cys Thr Ser Ile Cys Ser Leu Tyr Gln Leu
1               5                   10                  15

Glu Asn Tyr Cys Asn
            20

<210>  18
<211>  29
<212>  PRT
<213>  Unknown

<220>
<223>  Artificial Sequence

<220>
<221>  MISC_FEATURE
<222>  (29)..(29)
<223>  myristic acid is added to the epsilon-amino group of K in
       position 29

<400>  18

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys
            20                  25

<210>  19
<211>  21

<212>  PRT
<213>  Unknown

<220>
<223>  Artificial Sequence

<400>  19

Gly Ile Val Glu Gln Cys Cys Thr Ser Ile Cys Ser Leu Tyr Gln Leu
1               5                   10                  15

Glu Asn Tyr Cys Asn
            20

<210>  20
<211>  29
<212>  PRT
<213>  Unknown

<220>
<223>  Artificial Sequence

<220>
<221>  MISC_FEATURE
<222>  (29)..(29)
<223>  16-carbon fatty acid is added to the epsilon-amino group of K in
       position 29 of chain B via a gamma-glutamic acid linker

<400>  20

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys
            20                  25

<210>  21
<211>  8
<212>  PRT
<213>  Unknown

<220>
<223>  Artificial Sequence

<400>  21

Asp Tyr Lys Asp Asp Asp Asp Lys
1               5

<210>  22
<211>  13
<212>  PRT
<213>  Unknown

<220>
<223>  Artificial Sequence

<400>  22

Asp Tyr Lys Asp His Asp Gly Asp Tyr Lys Asp His Asp
1               5                   10

<210>  23
<211>  22
<212>  PRT
<213>  Unknown

<220>
<223>  Artificial Sequence

<400>  23

Asp Tyr Lys Asp His Asp Gly Asp Tyr Lys Asp His Asp Ile Asp Tyr
1               5                   10                  15

Lys Asp Asp Asp Asp Lys
            20

<210>  24
<211>  51
<212>  PRT
<213>  Unknown

<220>
<223>  Artificial Sequence

<400>  24

Thr Asn Pro Gly Val Ser Ala Trp Gln Val Asn Thr Ala Tyr Thr Ala
1               5                   10                  15

Gly Gln Leu Val Thr Tyr Asn Gly Lys Thr Tyr Lys Cys Leu Gln Pro
            20                  25                  30

His Thr Ser Leu Ala Gly Trp Glu Pro Ser Asn Val Pro Ala Leu Trp
        35                  40                  45

Gln Leu Gln
    50

<210>  25
<211>  387
<212>  PRT
<213>  Unknown

<220>
<223>  Artificial Sequence

<400>  25

Met Lys Ile Glu Glu Gly Lys Leu Val Ile Trp Ile Asn Gly Asp Lys
1               5                   10                  15

Gly Tyr Asn Gly Leu Ala Glu Val Gly Lys Lys Phe Glu Lys Asp Thr
            20                  25                  30

Gly Ile Lys Val Thr Val Glu His Pro Asp Lys Leu Glu Glu Lys Phe
        35                  40                  45

Pro Gln Val Ala Ala Thr Gly Asp Gly Pro Asp Ile Ile Phe Trp Ala
    50                  55                  60

His Asp Arg Phe Gly Gly Tyr Ala Gln Ser Gly Leu Leu Ala Glu Ile
65                  70                  75                  80

Thr Pro Asp Lys Ala Phe Gln Asp Lys Leu Tyr Pro Phe Thr Trp Asp
                85                  90                  95

Ala Val Arg Tyr Asn Gly Lys Leu Ile Ala Tyr Pro Ile Ala Val Glu
            100                 105                 110

Ala Leu Ser Leu Ile Tyr Asn Lys Asp Leu Leu Pro Asn Pro Pro Lys
        115                 120                 125

Thr Trp Glu Glu Ile Pro Ala Leu Asp Lys Glu Leu Lys Ala Lys Gly
    130                 135                 140

Lys Ser Ala Leu Met Phe Asn Leu Gln Glu Pro Tyr Phe Thr Trp Pro
145                 150                 155                 160

Leu Ile Ala Ala Asp Gly Gly Tyr Ala Phe Lys Tyr Glu Asn Gly Lys
                165                 170                 175

Tyr Asp Ile Lys Asp Val Gly Val Asp Asn Ala Gly Ala Lys Ala Gly
            180                 185                 190

Leu Thr Phe Leu Val Asp Leu Ile Lys Asn Lys His Met Asn Ala Asp
        195                 200                 205

Thr Asp Tyr Ser Ile Ala Glu Ala Ala Phe Asn Lys Gly Glu Thr Ala
    210                 215                 220

Met Thr Ile Asn Gly Pro Trp Ala Trp Ser Asn Ile Asp Thr Ser Lys
225                 230                 235                 240

Val Asn Tyr Gly Val Thr Val Leu Pro Thr Phe Lys Gly Gln Pro Ser
                245                 250                 255

Lys Pro Phe Val Gly Val Leu Ser Ala Gly Ile Asn Ala Ala Ser Pro
            260                 265                 270

Asn Lys Glu Leu Ala Lys Glu Phe Leu Glu Asn Tyr Leu Leu Thr Asp
        275                 280                 285

Glu Gly Leu Glu Ala Val Asn Lys Asp Lys Pro Leu Gly Ala Val Ala
    290                 295                 300

Leu Lys Ser Tyr Glu Glu Glu Leu Ala Lys Asp Pro Arg Ile Ala Ala
305                 310                 315                 320

Thr Met Glu Asn Ala Gln Lys Gly Glu Ile Met Pro Asn Ile Pro Gln
                325                 330                 335

Met Ser Ala Phe Trp Tyr Ala Val Arg Thr Ala Val Ile Asn Ala Ala
            340                 345                 350

Ser Gly Arg Gln Thr Val Asp Glu Ala Leu Lys Asp Ala Gln Thr Asn
        355                 360                 365

Ser Ser Ser Asn Asn Asn Asn Asn Asn Asn Asn Asn Asn Leu Gly Ile
    370                 375                 380

Glu Gly Arg
385

<210>  26
<211>  10
<212>  PRT
<213>  Unknown

<220>
<223>  Artificial Sequence

<400>  26

Glu Gln Lys Leu Ile Ser Glu Glu Asp Leu
1               5                   10

<210>  27
<211>  38
<212>  PRT
<213>  Unknown

<220>
<223>  Artificial Sequence

<400>  27

Met Asp Glu Lys Thr Thr Gly Trp Arg Gly Gly His Val Val Glu Gly
1               5                   10                  15

Leu Ala Gly Glu Leu Glu Gln Leu Arg Ala Arg Leu Glu His His Pro

20                  25                  30

Gln Gly Gln Arg Glu Pro
        35

<210>  28
<211>  15
<212>  PRT
<213>  Unknown

<220>
<223>  Artificial Sequence

<400>  28

Lys Glu Thr Ala Ala Ala Lys Phe Glu Arg Gln His Met Asp Ser
1               5                   10                  15

<210>  29
<211>  19
<212>  PRT
<213>  Unknown

<220>
<223>  Artificial Sequence

<400>  29

Lys Asp His Leu Ile His Asn Val His Lys Glu Phe His Ala His Ala
1               5                   10                  15

His Asn Lys

<210>  30
<211>  26
<212>  PRT
<213>  Unknown

<220>
<223>  Artificial Sequence

<400>  30

Lys Arg Arg Trp Lys Lys Asn Phe Ile Ala Val Ser Ala Ala Asn Arg
1               5                   10                  15

Phe Lys Lys Ile Ser Ser Ser Gly Ala Leu
            20                  25

<210>  31
<211>  8
<212>  PRT
<213>  Unknown

<220>

<223>  Artificial Sequence

<400>  31

Trp Ser His Pro Gln Phe Glu Lys
1               5


<210>  32
<211>  232
<212>  PRT
<213>  Unknown

<220>
<223>  Artificial Sequence

<400>  32

Met Ser Pro Ile Leu Gly Tyr Trp Lys Ile Lys Gly Leu Val Gln Pro
1               5                   10                  15

Thr Arg Leu Leu Leu Glu Tyr Leu Glu Glu Lys Tyr Glu Glu His Leu
            20                  25                  30

Tyr Glu Arg Asp Glu Gly Asp Lys Trp Arg Asn Lys Lys Phe Glu Leu
        35                  40                  45

Gly Leu Glu Phe Pro Asn Leu Pro Tyr Tyr Ile Asp Gly Asp Val Lys
    50                  55                  60

Leu Thr Gln Ser Met Ala Ile Ile Arg Tyr Ile Ala Asp Lys His Asn
65                  70                  75                  80

Met Leu Gly Gly Cys Pro Lys Glu Arg Ala Glu Ile Ser Met Leu Glu
                85                  90                  95

Gly Ala Val Leu Asp Ile Arg Tyr Gly Val Ser Arg Ile Ala Tyr Ser
            100                 105                 110

Lys Asp Phe Glu Thr Leu Lys Val Asp Phe Leu Ser Lys Leu Pro Glu
        115                 120                 125

Met Leu Lys Met Phe Glu Asp Arg Leu Cys His Lys Thr Tyr Leu Asn
    130                 135                 140

Gly Asp His Val Thr His Pro Asp Phe Met Leu Tyr Asp Ala Leu Asp
145                 150                 155                 160

Val Val Leu Tyr Met Asp Pro Met Cys Leu Asp Ala Phe Pro Lys Leu
                165                 170                 175

Val Cys Phe Lys Lys Arg Ile Glu Ala Ile Pro Gln Ile Asp Lys Tyr

            180                 185                 190

Leu Lys Ser Ser Lys Tyr Ile Ala Trp Pro Leu Gln Gly Trp Gln Ala
        195                 200                 205

Thr Phe Gly Gly Gly Asp His Pro Pro Lys Ser Asp Leu Val Pro Arg
    210                 215                 220

Gly Ser Pro Gly Ile His Arg Asp
225                 230

<210> 33
<211> 6
<212> PRT
<213> Unknown

<220>
<223> Artificial Sequence

<400> 33

His His His His His His
1               5

<210> 34
<211> 122
<212> PRT
<213> Unknown

<220>
<223> Artificial Sequence

<400> 34

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Pro Asp Tyr Lys Asp Asp Asp Asp Lys
        115                 120

<210>  35
<211>  123
<212>  PRT
<213>  Unknown

<220>
<223>  Artificial Sequence

<400>  35

Met Asp Tyr Lys Asp Asp Asp Asp Lys Met Thr Cys Lys Met Ser Gln
1               5                   10                  15

Leu Glu Arg Asn Ile Glu Thr Ile Ile Asn Thr Phe His Gln Tyr Ser
            20                  25                  30

Val Lys Leu Gly His Pro Asp Thr Leu Asn Gln Gly Glu Phe Lys Glu
        35                  40                  45

Leu Val Arg Lys Asp Leu Gln Asn Phe Leu Lys Lys Glu Asn Lys Asn
    50                  55                  60

Glu Lys Val Ile Glu His Ile Met Glu Asp Leu Asp Thr Asn Ala Asp
65                  70                  75                  80

Lys Gln Leu Ser Phe Glu Glu Phe Ile Met Leu Met Ala Arg Leu Thr
                85                  90                  95

Trp Ala Ser His Glu Lys Met His Glu Gly Asp Glu Gly Pro Gly His
            100                 105                 110

His His Lys Pro Gly Leu Gly Glu Gly Thr Pro
        115                 120

<210>  36
<211>  99
<212>  PRT
<213>  Unknown

<220>
<223>  Artificial Sequence

<400>  36

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His Asp Tyr Lys Asp Asp
                85                  90                  95

Asp Asp Lys

<210>  37
<211>  100
<212>  PRT
<213>  Unknown

<220>
<223>  Artificial Sequence

<400>  37

Met Asp Tyr Lys Asp Asp Asp Asp Lys Met Thr Cys Lys Met Ser Gln
1               5                   10                  15

Leu Glu Arg Asn Ile Glu Thr Ile Ile Asn Thr Phe His Gln Tyr Ser
            20                  25                  30

Val Lys Leu Gly His Pro Asp Thr Leu Asn Gln Gly Glu Phe Lys Glu
        35                  40                  45

Leu Val Arg Lys Asp Leu Gln Asn Phe Leu Lys Lys Glu Asn Lys Asn
    50                  55                  60

Glu Lys Val Ile Glu His Ile Met Glu Asp Leu Asp Thr Asn Ala Asp
65                  70                  75                  80

Lys Gln Leu Ser Phe Glu Glu Phe Ile Met Leu Met Ala Arg Leu Thr
                85                  90                  95

Trp Ala Ser His
            100

<210>  38
<211>  84
<212>  PRT
<213>  Unknown

<220>
<223>  Artificial Sequence

<400>  38

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Asp Tyr Lys Asp
65                  70                  75                  80

Asp Asp Asp Lys

<210>  39
<211>  85
<212>  PRT
<213>  Unknown

<220>
<223>  Artificial Sequence

<400>  39

Met Asp Tyr Lys Asp Asp Asp Asp Lys Met Thr Cys Lys Met Ser Gln
1               5                   10                  15

Leu Glu Arg Asn Ile Glu Thr Ile Ile Asn Thr Phe His Gln Tyr Ser
            20                  25                  30

Val Lys Leu Gly His Pro Asp Thr Leu Asn Gln Gly Glu Phe Lys Glu
        35                  40                  45

Leu Val Arg Lys Asp Leu Gln Asn Phe Leu Lys Lys Glu Asn Lys Asn
    50                  55                  60

Glu Lys Val Ile Glu His Ile Met Glu Asp Leu Asp Thr Asn Ala Asp
65                  70                  75                  80

Lys Gln Leu Ser Phe
                85

<210>  40
<211>  100
<212>  PRT
<213>  Unknown

<220>
<223>  Artificial Sequence

<400>  40

Met Asp Tyr Lys Asp Asp Asp Asp Lys Met Lys Leu Gly His Pro Asp
1               5                   10                  15

Thr Leu Asn Gln Gly Glu Phe Lys Glu Leu Val Arg Lys Asp Leu Gln
            20                  25                  30

Asn Phe Leu Lys Lys Glu Asn Lys Asn Glu Lys Val Ile Glu His Ile
        35                  40                  45

Met Glu Asp Leu Asp Thr Asn Ala Asp Lys Gln Leu Ser Phe Glu Glu
    50                  55                  60

Phe Ile Met Leu Met Ala Arg Leu Thr Trp Ala Ser His Glu Lys Met
65                  70                  75                  80

His Glu Gly Asp Glu Gly Pro Gly His His His Lys Pro Gly Leu Gly
                85                  90                  95

Glu Gly Thr Pro
            100

<210>  41
<211>  99
<212>  PRT
<213>  Unknown

<220>
<223>  Artificial Sequence

<400>  41

Met Lys Leu Gly His Pro Asp Thr Leu Asn Gln Gly Glu Phe Lys Glu
1               5                   10                  15

Leu Val Arg Lys Asp Leu Gln Asn Phe Leu Lys Lys Glu Asn Lys Asn
            20                  25                  30

Glu Lys Val Ile Glu His Ile Met Glu Asp Leu Asp Thr Asn Ala Asp
        35                  40                  45

Lys Gln Leu Ser Phe Glu Glu Phe Ile Met Leu Met Ala Arg Leu Thr
    50                  55                  60

Trp Ala Ser His Glu Lys Met His Glu Gly Asp Glu Gly Pro Gly His
65                  70                  75                  80

His His Lys Pro Gly Leu Gly Glu Gly Thr Pro Asp Tyr Lys Asp Asp
                85                  90                  95

Asp Asp Lys

<210>  42
<211>  84
<212>  PRT
<213>  Unknown

<220>
<223>  Artificial Sequence

<400>  42

Met Leu Val Arg Lys Asp Leu Gln Asn Phe Leu Lys Lys Glu Asn Lys
1               5                   10                  15

Asn Glu Lys Val Ile Glu His Ile Met Glu Asp Leu Asp Thr Asn Ala
            20                  25                  30

Asp Lys Gln Leu Ser Phe Glu Glu Phe Ile Met Leu Met Ala Arg Leu
        35                  40                  45

Thr Trp Ala Ser His Glu Lys Met His Glu Gly Asp Glu Gly Pro Gly
    50                  55                  60

His His His Lys Pro Gly Leu Gly Glu Gly Thr Pro Asp Tyr Lys Asp
65                  70                  75                  80

Asp Asp Asp Lys

<210>  43
<211>  85
<212>  PRT
<213>  Unknown

<220>
<223>  Artificial Sequence

<400>  43

Met Asp Tyr Lys Asp Asp Asp Asp Lys Met Leu Val Arg Lys Asp Leu

1               5                   10                  15

Gln Asn Phe Leu Lys Lys Glu Asn Lys Asn Glu Lys Val Ile Glu His
            20                  25                  30

Ile Met Glu Asp Leu Asp Thr Asn Ala Asp Lys Gln Leu Ser Phe Glu
        35                  40                  45

Glu Phe Ile Met Leu Met Ala Arg Leu Thr Trp Ala Ser His Glu Lys
    50                  55                  60

Met His Glu Gly Asp Glu Gly Pro Gly His His His Lys Pro Gly Leu
65                  70                  75                  80

Gly Glu Gly Thr Pro
                85

<210>  44
<211>  122
<212>  PRT
<213>  Unknown

<220>
<223>  Artificial Sequence

<400>  44

Met Thr Cys Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Ala Ala Asp Lys Gln Leu Ser Phe Glu Ala Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Pro Asp Tyr Lys Asp Asp Asp Asp Lys
        115                 120

<210> 45
<211> 393
<212> DNA
<213> Unknown

<220>
<223> Artificial Sequence

<400> 45
gctagcggat ccgccgccac catggccaac aaagcacctt ctcagatgga gcgcagcata 60

accaccatca tcgacacctt ccatcaatac tctaggaagg aaggacaccc tgacaccctg 120

agcaagaagg aattcagaca aatggtggaa gcacagttgg caacctttat gaagaaagag 180

aagagaaatg aagccctcat aaatgacatc atggaggacc tggacacaaa ccaggacaat 240

cagctgagct ttgaggagtg tatgatgctg atggcaaagt tgatctttgc ctgtcatgag 300

aagctgcatg agaacaaccc acgtgggcat ggccacagtc atggcaaagg ctgtgggaag 360

gactacaaag acgatgacga caagtgactc gag 393

<210> 46
<211> 114
<212> PRT
<213> Unknown

<220>
<223> Artificial Sequence

<400> 46

Met Thr Ser Lys Met Ser Gln Leu Glu Arg Asn Ile Glu Thr Ile Ile
1               5                   10                  15

Asn Thr Phe His Gln Tyr Ser Val Lys Leu Gly His Pro Asp Thr Leu
            20                  25                  30

Asn Gln Gly Glu Phe Lys Glu Leu Val Arg Lys Asp Leu Gln Asn Phe
        35                  40                  45

Leu Lys Lys Glu Asn Lys Asn Glu Lys Val Ile Glu His Ile Met Glu
    50                  55                  60

Asp Leu Asp Thr Asn Ala Asp Lys Gln Leu Ser Phe Glu Glu Phe Ile
65                  70                  75                  80

Met Leu Met Ala Arg Leu Thr Trp Ala Ser His Glu Lys Met His Glu
                85                  90                  95

Gly Asp Glu Gly Pro Gly His His His Lys Pro Gly Leu Gly Glu Gly
            100                 105                 110

Thr Cys

<210> 47
<211> 16
<212> PRT
<213> Unknown

<220>
<223> Artificial Sequence

<400> 47

Gly Ser Ser Gly Ser Ser Gly Ser Ser Gly Ser Ser Gly Ser Ser Gly
1               5                   10                  15

<210> 48
<211> 15
<212> PRT
<213> Unknown

<220>
<223> Artificial Sequence

<400> 48

Gly Leu Asn Asp Ile Phe Glu Ala Gln Lys Ile Glu Trp His Glu
1               5                   10                  15

## Description

### FIELD

The present disclosure provides compositions and methods for their use in the treatment of an insulin deficiency (ID) condition, or an associated symptom, in a subject in need thereof, the compositions comprising a S100 calcium-binding protein A9 (S100A9), a variant or a fragment thereof and insulin, a variant or a fragment thereof.

### BACKGROUND OF THE INVENTION

Tens of millions suffer from type 1 diabetes (T1D); a condition caused by an autoimmune-mediated attack of pancreatic β-cells leading to total (or almost total) β-cell loss and insulin deficiency 1. If untreated, T1D is a lethal catabolic disease characterized by hyperglycemia. Thus, the focus of T1D research and drug development has been mainly on improving strategies to lower hyperglycemia without causing life-threatening hypoglycemia ^{2,3}. However, in addition to increased circulating glucose level β-cell loss leads to several "other defects", some of which (e.g. severe hyperketonemia and ketoacidosis) are life-threatening⁴⁻⁷. Therefore, it is important to develop strategies that in addition to improve hyperglycemia can also rescue the "other defects" (e.g. increased ketogenesis) caused by insulin deficiency. For example, our unpublished results shown below underscore the importance of ameliorating hyperglycemia and the "other defects". Indeed, our data indicate that despite the presence of slightly improved hyperglycemia a normalization of hyperketonemia and hypertriglyceridemia is associated with a significant extension in lifespan of mice with β-cell loss and insulin deficiency.

Untreated T1D rapidly leads to death 4. However, since insulin was discovered in the early 1920s ^{8,9}, T1D has been treated with insulin therapy; an approach that converted this lethal disease into one a person can live with. The remarkable achievement of insulin (which represents one of the most important discoveries in medicine) led to the conclusion that life without insulin is not possible, nevertheless the scientific community has to acknowledge that insulin therapy is unsatisfactory4. Indeed, T1D subjects have higher risks for developing kidney failure, blindness, nerve damage, heart attack, stroke, and hypoglycemia⁴. Some of these defects may be favored by insulin therapy itself. For example, insulin stimulates lipid and cholesterol synthesis; thus, probably owing to its established lipogenic actions¹⁰ chronic insulin therapy promotes lipid deposition outside adipose tissue. This effect could contribute to the extremely high incidence of coronary artery disease observed in diabetic subjects ^{5,6}. In addition, the lipogenic actions of insulin promote lipid-induced insulin resistance and therefore could underlie, at least in part, the increased insulin needs in long-term T1D care ¹¹. Insulin is also a potent glycemia-lowering hormone. Owing to this action, intensive insulin therapy causes hypoglycemia that can be disabling and sometimes could lead to death ¹²⁻¹⁴. Because insulin therapy does not eradicate the disabling co-morbidities of T1D (e.g. heart attack, stroke, blindness, kidney failure, neuropathy, etc.) the costs needed for T1D care are immense and the quality of life of T1D patients is reduced compared to normal subjects 15. Due to the shortcoming of current treatment, research aimed at improving T1D therapy is urgently needed.

The main approach is aiming at diminishing the amount of insulin dosage and hence reducing risks associated with insulin therapy as for example life-threatening hypoglycemia. Yet, virtually all the prospected adjunct treatments to insulin focus on improving hyperglycemia. For example, the synthetic analog of amylin (pramlintide), incretin mimetics (e.g. glucagon-like-peptide-1 receptor agonists and dipeptidyl-peptidase-4 inhibitors), and sodium-glucose-transporter-1 and -2 (SGLT1 and 2) inhibitors aim at lowering hyperglycemia and are associated with increased risks of hypoglycemia ^{2,3}. Some of these therapies are also associated with increased risks of ketoacidosis ^{2,3}.

Therefore, there remains a need for improved therapeutic methods for treating insulin deficiency (ID) condition, or an associated symptom, in a subject in need thereof which reduces the risks of hypoglycemia and ketoacidosis.

### SUMMARY OF THE INVENTION

The present invention provides a composition for use in the treatment of insulin deficiency (ID) condition, or an associated symptom, in a subject in need thereof, the composition comprising
i) a S100 calcium-binding protein A9 (S100A9), a variant or a fragment thereof and
ii) insulin, a variant or a fragment thereof.

Another aspect of the invention concerns a method of treating an insulin deficiency (ID) condition, or an associated symptom, in a subject in need thereof, the method comprising administering to the subject a therapeutically-effective amount of
i) a S100 calcium-binding protein A9 (S100A9), a variant or a fragment thereof and
ii) insulin, a variant or a fragment thereof.

### DESCRIPTION OF THE FIGURES

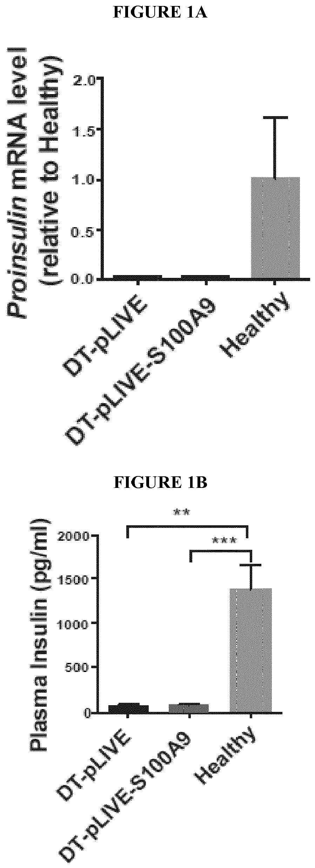

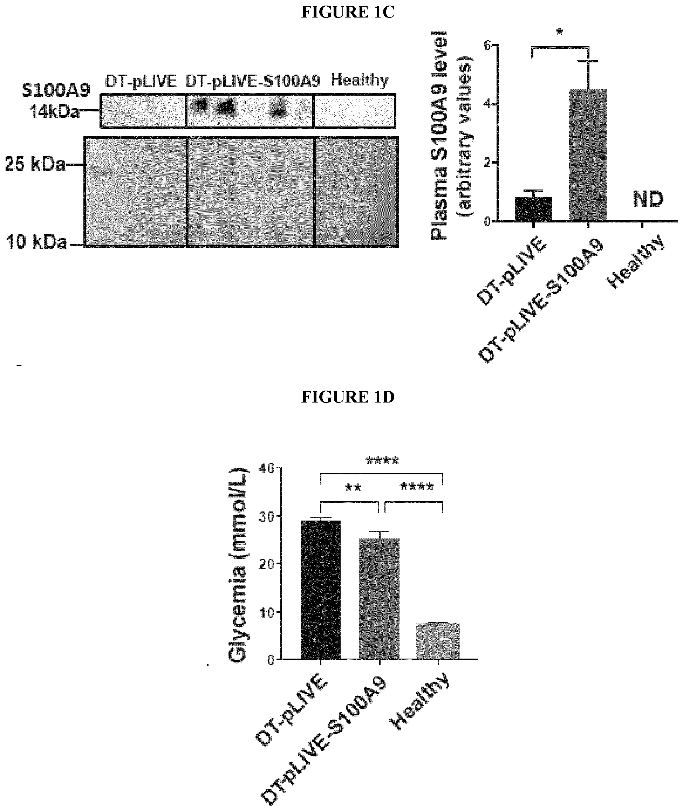

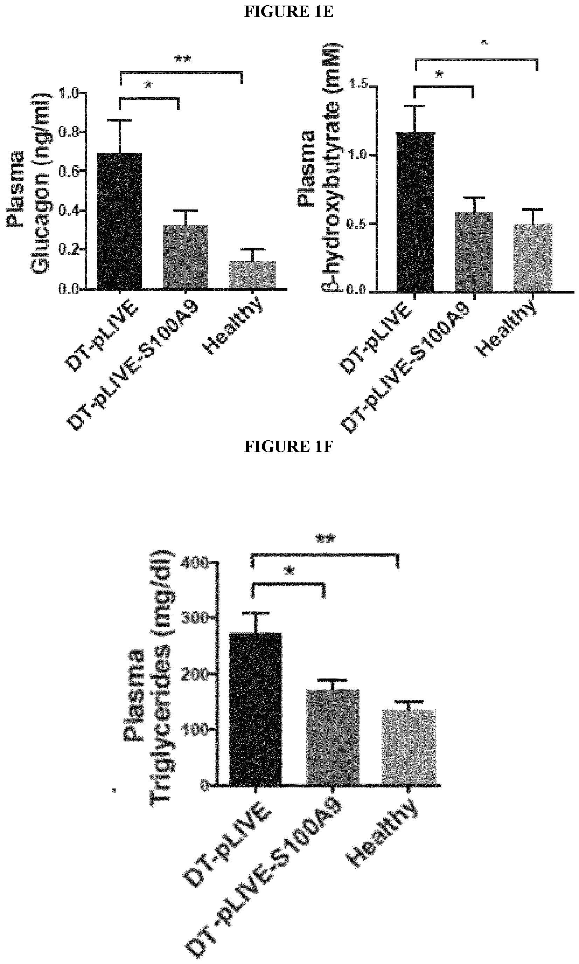

**Figure 1****. S100A9 ameliorates metabolic imbalance in DT-induced ID mice**. (**a**) *Proinsulin* mRNA content in DT-treated *RIP-DTR* mice (sacrificed 10 days after hydrodynamic tail vein injection; HTVI) and their age-matched non-diabetic healthy controls, (**b**) Plasma insulin content in DT-pLIVE and DT-pLIVE-S100A9 mice (10 days after HTVI) and age-matched healthy controls. (**c**) Plasmatic S100A9 levels, and circulating (**d**) glucose, (**e**) glucagon and β-hydroxybutyrate, and (**f**) triglycerides. Error bars represent SEM. Statistical analyses were done using one-way ANOVA (Tukey's post-hoc test). Healthy (n = 3-6), DT-pLIVE (n = 7-12) and DT-pLIVE-S100A9 (n = 7-12). *P < 0.05; **P < 0.01; ***P < 0.001, ****P < 0.0001.

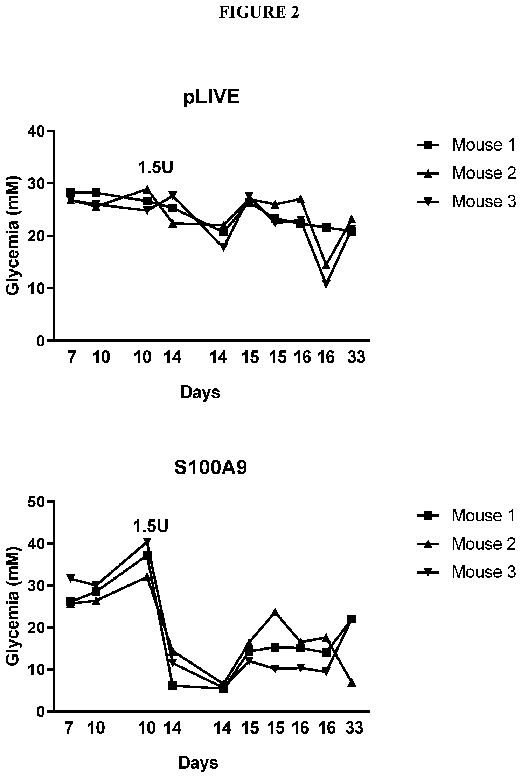

**Figure 2****. Enhanced S100A9 ameliorates insulin effectiveness in lowering hyperglycemia in ID mice.** The insulin dose at 1.5U/mouse did not affect hyperglycemia in DT-pLIVE mice (14 days after hydrodynamic tail vein injection; (HTVI)) while it did cause a rapid decrease in hyperglycemia in DT-pLIVE-S100A9 mice (14 days after HTVI). Glycemia was measured 3 hours after the insulin injection.

### DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting.

In the case of conflict, the present specification, including definitions, will control. Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise/comprising" is generally used in the sense of include/including, that is to say permitting the presence of one or more features or components.

As used in the specification and claims, the singular form "a", "an" and "the" include plural references unless the context clearly dictates otherwise.

As used herein, "at least one" means "one or more", "two or more", "three or more", etc.

As used herein the terms "subject", "subject in need thereof', or "patient", "patient in need thereof " are well-recognized in the art, and, are used interchangeably herein to refer to a mammal, including dog, cat, rat, mouse, monkey, cow, horse, goat, sheep, pig, camel, and, most preferably, a human. In some cases, the subject is a subject in need of treatment or a subject with a disease or disorder. However, in other aspects, the subject can be a normal subject. The term does not denote a particular age or sex. Thus, adult and newborn subjects, whether male or female, are intended to be covered. Preferably, the subject is a human, most preferably a human suffering from insulin deficiency (ID) condition, or an associated symptom.

The term "treating", "treated" or "treatment" as used herein includes preventative (e.g. prophylactic), palliative, and curative uses or results.

The term "insulin deficiency" as used herein refers to a partial or complete loss of pancreatic insulin-producing beta-cells. The term further includes their reduced capacity of secreting insulin resulting in reduced level of circulating insulin.

The term "insulin deficiency associated symptom" as used herein refers to adverse effect(s) caused by low or absent levels of insulin.

Insulin deficiency associated symptom is selected from the group comprising hyperglycemia, hyperketonemia, ketoacidosis, hypertriglyceridemia, hyperglucagonemia, hypercalprotectinemia, increased or high circulating (non-esterified fatty acids (NEFAs) level, severe hypoleptinemia, reduced or low body fat mass, hyperphagia, polydipsia and any combination thereof.

Insulin deficiency (ID) condition usually refers to diabetes type 1 or to sub-types of diabetes type 2.

The terms "nucleic acid", "polynucleotide", and "oligonucleotide" are used interchangeably and refer to any kind of deoxyribonucleotide (e.g. DNA, cDNA, ...) or ribonucleotide (e.g. RNA, mPvNA, ...) polymer or a combination of deoxyribonucleotide and ribonucleotide (e.g. DNA/RNA) polymer, in linear or circular conformation, and in either single - or double - stranded form. These terms are not to be construed as limiting with respect to the length of a polymer and can encompass known analogues of natural nucleotides, as well as nucleotides that are modified in the base, sugar and/or phosphate moieties (e.g. phosphorothioate backbones). In general, an analogue of a particular nucleotide has the same base-pairing specificity; i.e., an analogue of A will base-pair with T.

The term "vector", as used herein, refers to a viral vector or to a nucleic acid (DNA or RNA) molecule such as a plasmid or other vehicle, which contains one or more heterologous nucleic acid sequence(s) (such as nucleic acid sequence(s) encoding the one or more nucleic acid(s) encoding the peptides (e.g. S100A9, Insulin, and/or Affinity Tag, variants or fragments thereof, of the invention). The terms "expression vector", "gene delivery vector" and "gene therapy vector" refer to any vector that is effective to incorporate and express one or more nucleic acid(s), in a cell, preferably under the regulation of a promoter. A cloning or expression vector may comprise additional elements, for example, regulatory and/or post-transcriptional regulatory elements in addition to a promoter.

The term "about," particularly in reference to a given quantity, is meant to encompass deviations of plus or minus ten (10) percent.

As used herein, a "fragment" of a protein, peptide or polypeptide of the invention refers to a sequence containing less amino acids in length than the protein, peptide or polypeptide of the invention. This sequence can be used as long as it exhibits the same properties, i.e is biologically active, as the native sequence from which it derives.

The term "variant" refers to a protein, peptide or polypeptide having an amino acid sequence that differ to some extent from a native sequence peptide, that is an amino acid sequence that vary from the native sequence by amino acid substitutions, whereby one or more amino acids are substituted by another with same characteristics and conformational roles. The amino acid sequence variants possess substitutions, deletions, and/or insertions at certain positions within the amino acid sequence of the native amino acid sequence. Substitutions can also be conservative, in this case, the conservative amino acid substitutions are herein defined as exchanges within one of the following five groups:
I. Small aliphatic, nonpolar or slightly polar residues: Ala, Ser, Thr, Pro, Gly
II. Polar, positively charged residues: His, Arg, Lys
III. Polar, negatively charged residues: and their amides: Asp, Asn, Glu, Gin
IV. Large, aromatic residues: Phe, Tyr, Tip
V. Large, aliphatic, nonpolar residues: Met, Leu, Ile, Val, Cys.

The present invention is based, in part, on the surprising finding that the administration of S100 calcium-binding protein A9 (S100A9) along with an otherwise sub-optimal insulin dose greatly improves metabolism of insulin deficient mice without causing hypoglycaemia.

S100A9, that belongs to the EF-hand superfamily of Ca2+-binding proteins, is highly expressed in monocytes and neutrophils and secreted in conditions of elevated inflammation as for example in rheumatoid arthritis or sepsis 16,17. With its partner S100A8, S100A9 forms a heterocomplex (S100A9/S100A8; also known as calprotectin) that is also secreted in response to inflammatory states18. Calprotectin is an endogenous activator of the Toll-like receptor 4 (TLR4)17 and the receptor of advanced glycated end-products (RAGE)19. Calprotectin has been shown to exert several deleterious effects including underlying sepsis-induced lethality ^{16,17,19-21}. However, others have shown that calgranulins can also exist in monomers to exert anti-inflammatory effects20. Noteworthy, S100A9 homodimers have been reported to directly affect TLR4 signaling22. Collectively, these data indicate that calprotectin (S100A9/S100A8 heterodimer) and S100A9 (S100A9/S100A9 homodimer) regulate inflammatory pathways. Although calprotectin is considered to exert deleterious effects, there is murine and human evidence indicating that S100A9 is beneficial. For example, enhanced S100A9 brings about significant beneficial metabolic effects in T1D mice (Fig. 1).

An aspect of the present invention concerns a composition for use in the treatment of an insulin deficiency (ID) condition, or an associated symptom, in a subject in need thereof, the composition comprising a
i) S100 calcium-binding protein A9 (S100A9), a variant or a fragment thereof and
ii) insulin, a variant or a fragment thereof.

The treatment comprises alleviating hyperglycemia, alleviating and/or reducing risk of hypoglycemia, alleviating increased level of glycated hemoglobin in the blood, alleviating hyperglucagonemia, alleviating and/or reducing risk of hyperketonemia and ketoacidosis, alleviating hypertriglyceridemia, alleviating increased hepatic fatty acid oxidation (FAO), increasing hepatic native or modified S100A9 mRNA level, increasing hepatic native or modified S100A9 protein level, increasing plasmatic native or modified S100A9 protein level, increasing hepatic ATP level, increasing lifespan, decreasing circulating non-esterified fatty acids (NEFAs) level, decreasing hepatic mitochondrial DNA level, decreasing circulating calprotectin level, decreasing lipase activity, or any combination thereof.

In certain aspects, the treatment comprises decreasing the insulin dose, or the variant or fragment thereof, by at least 5%, by at least 10%, by at least 15%, by at least 20%, by at least 25%, by at least 30%, by at least 35%, by at least 40%, by at least 45%, by at least 50%, or more as compared to the administration of insulin in the absence of a S100A9 protein, a variant or a fragment thereof.

Preferably, the S100A9 protein is a native or recombinant protein having an amino-acid sequence as set forth in SEQ ID NO: 1, a variant or a fragment thereof.

A fragment of the S100A9 protein is preferably an active fragment comprising at least about 25 consecutive amino-acids, at least about 30 consecutive amino-acids, at least about 35 consecutive amino-acids, at least about 40 consecutive amino-acids, at least about 50 consecutive amino-acids, at least about 60 consecutive amino-acids, at least about 67 consecutive amino-acids, at least about 70 consecutive amino-acids, at least about 75 consecutive amino-acids, at least about 80 consecutive amino-acids, at least about 85 consecutive amino-acids, at least about 91 consecutive amino-acids, at least about 95 consecutive amino-acids, at least about 100 consecutive amino-acids, at least about 105 consecutive amino-acids, at least about 110 consecutive amino-acids, at least about 115 consecutive amino-acids, at least about 120 consecutive amino-acids, at least about 125 consecutive amino-acids or at least about 130 consecutive amino-acids of the amino-acid sequence set forth in SEQ ID NO: 1.

Non-limiting examples of S100A9 fragments comprise S100A9 N91 (SEQ ID NO: 2), S100A9 C91 (SEQ ID NO: 3), S100A9 N76 (SEQ ID NO: 4) and S100A9 C76 (SEQ ID NO: 5), and a combination of one more thereof.

A variant of the S100A9 protein differs from the amino-acid sequence set forth in SEQ ID NO: 1, or from an active fragment thereof, in 1 to about 10 amino acids. Preferably, the amino acid sequence variants possess substitutions, deletions at the N- and/or C-terminus, as well as within one or more internal domains, and/or insertions at certain positions within the amino acid sequence of the native amino acid sequence as described above.

Both the variant and fragment of the S100A9 protein can include synthetic, non-standard and/or naturally-occurring amino acid sequences (including D-forms and/or retro-inverso isomers) derivable from the naturally occurring amino acid sequence of the S100A9 protein. By way of example, the replacement amino acid may be a basic non-standard amino acid, (e.g. L-Ornithine, L-2-amino-3-guanidinopropionic acid, or D-isomers of Lysine, Arginine and Ornithine). Methods for introducing non-standard amino acids into proteins are known in the art, and include recombinant protein synthesis using E. coli auxotrophic expression hosts.

Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins.

An examples of an S100A9 variant comprise the S100A9 N69A-E78A variant having an amino-acid sequence as set forth in SEQ ID NO: 6.

The S100A9 protein, variant or fragment thereof may also be conjugated to a chemical or enzymatic moiety. These moieties are typically used to increase solubility, prolong stability, reduce immunogenicity and/or enable fusion with an immunoglobulin or a particular region of an immunoglobulin. Non-limiting examples of these moieties comprise PEG, Maleimide-PEG(n)-succinimidyl ester and biotin.

Insulin is selected from native insulin, recombinant insulin, proinsulin, basal insulin or bolus insulin. Insulin is a protein comprising a chain A and a chain B having an amino-acid sequence as set forth in, respectively SEQ ID NO: 7 or SEQ ID NO: 8, a variant or a fragment thereof.

A fragment of insulin protein refers to a fragment of chain A and/or chain B of insulin. Insulin fragments of A chain have an A chain length of at least about 15 consecutive amino-acids, at least about 16 consecutive amino-acids, at least about 17 consecutive amino-acids, at least about 18 consecutive amino-acids, at least about 19 consecutive amino-acids, at least about 20 consecutive amino-acids, at least about 21 consecutive amino-acids, at least about 22 consecutive amino-acids, at least about 23 consecutive amino-acids, at least about 24 consecutive amino-acids, at least about 25 consecutive amino-acids, at least about 26 consecutive amino-acids, at least about 27 consecutive amino-acids, at least about 28 consecutive amino-acids, at least about 29 consecutive amino-acids, at least about 30 consecutive amino-acids, at least about 35 consecutive amino-acids, or more of the native A chain amino acid sequence. Insulin fragments of B chain have a B chain length of at least about 25 consecutive amino-acids, at least about 26 consecutive amino-acids, at least about 27 consecutive amino-acids, at least about 28 consecutive amino-acids, at least about 29 consecutive amino-acids, at least about 29 consecutive amino-acids, at least about 30 consecutive amino-acids, at least about 31 consecutive amino-acids, at least about 32 consecutive amino-acids, at least about 33 consecutive amino-acids, at least about 34 consecutive amino-acids, at least about 35 consecutive amino-acids, at least about 36 consecutive amino-acids, at least about 37 consecutive amino-acids, at least about 38 consecutive amino-acids, at least about 39 consecutive amino-acids, at least about 40 consecutive amino-acids, at least about 41 consecutive amino-acids, at least about 42 consecutive amino-acids, at least about 42 consecutive amino-acids, at least about 43 consecutive amino-acids, at least about 44 consecutive amino-acids, at least about 45 consecutive amino-acids, at least about 50 consecutive amino-acids or more of the native amino acid B chain sequence.

A variant of the insulin protein differs from the amino-acid sequences set forth in SEQ IDs NO: 7 and/or 8, or from an active fragment thereof, in 1 to about 10 amino acids. Preferably, the amino acid sequence variants possess substitutions at the N- and/or C-terminus of at least one of the two chains, as well as within one or more internal domains, deletions, and/or insertions at certain positions within the amino acid sequence of the amino acid sequences as described above.

Non-limiting examples of insulin variants are selected from the group comprising insulin Lispro (SEQ IDs No. 9 and/or 10), insulin Glulisine (SEQ IDs No. 11 and/or 12), insulin Aspart (SEQ IDs No. 13 and/or 14), insulin Glargine (SEQ IDs No. 15 and/or 16), insulin Detemir (SEQ IDs No. 17 and/or 18), and insulin Deglutec (SEQ IDs No. 19 and/or 20), and a combination of one more thereof.

Both the variants and fragments of the insulin protein can include synthetic and/or naturally-occurring amino acid sequences (including D-forms and/or retro-inverso isomers) derivable from the naturally occurring amino acid sequence of the insulin protein and described above.

The insulin protein, variant or fragment thereof may also be conjugated to a chemical or enzymatic moiety. These moieties are typically used to increase solubility, prolong stability, reduce immunogenicity and/or enable fusion with an immunoglobulin or a particular region of an immunoglobulin. Non-limiting examples of these moieties comprise PEG and biotin.

In a certain aspect of the present invention, the S100A9 protein, variant or fragment thereof, further comprises at least one affinity tag.

An affinity tag is usually fused to either the C and/or N terminus of a recombinant protein to facilitate affinity purification and detection. This approach enables high selective capture and circumvents the multistep purification processes that limit throughput during R&D.

The affinity tag of the invention can be any molecule, peptide or not, useful in both research and therapy that can be added to the S100A9 protein, variant or fragment thereof (Kimple et al., in Curr Protoc Protein Sci. ; 73: Unit-9.9., 2013) and/or to the insulin, variant or fragment thereof.

Preferably, the affinity tag is selected from the group comprising FLAG tag (SEQ ID NO: 21), chitin binding protein (CBP) tag (SEQ ID NO: 24), maltose binding protein (MBP) tag (SEQ ID NO: 25), Strep tag II (SEQ ID NO: 31), glutathione-S-transferase (GST) tag (SEQ ID NO: 32), poly(His) tag (SEQ ID NO: 33), C-myc (SEQ ID NO: 26), SBP (SEQ ID NO: 27), S (SEQ ID NO: 28), HAT (SEQ ID NO: 29), and a combination of one more thereof.

More preferably, the affinity tag is a FLAG tag consisting or comprising the amino-acid sequence set forth in SEQ ID NO: 21 and a combination of one more thereof. Examples of combinations, or tandems, of the FLAG tag comprise 2x FLAG (SEQ ID NO: 22), 3x FLAG (SEQ ID NO: 23), etc...

Alternatively, the final tag of the 3x FLAG combination may encode an enterokinase cleavage site as set forth in SEQ ID NO: 23 (DYKDHD-G-DYKDHD-1-DYKDDDDK).

Non-limiting examples of S100A9 protein, variant or fragment thereof, that comprise one or more FLAG tag are selected from those listed in Table 1.

In a certain aspect of the invention, the i) S100A9 protein, variant or fragment thereof, ii) insulin, variant or fragment thereof, and, when present, the iii) at least one affinity tag are on the same peptide. Any combination can be envisioned, such as e.g. (from the N-terminus to the C-terminus): S100A9-Affinity Tag-Insulin, or S100A9- Insulin, or Insulin- S100A9, or Insulin - Affinity Tag-S100A9, or Affinity Tag-Insulin-S100A9, or Insulin-S100A9- Affinity Tag, or Affinity Tag-S100A9- Insulin, on the same peptide, separated or not by a peptidyl or non-peptidyl linker. An example of peptidyl linker (SEQ ID No. 47) is given in Table 1.

The composition of the invention may further comprising a sodium-glucose cotransporter 1 (SGLT1) and/or 2 (SGLT2) inhibitor(s), amylin analogs, biguanides (e.g., metformin), incretin mimetics (e.g., glucagon-like peptide receptor agonists, dipeptidyl-peptidase-4 inhibitors).

The present invention also contemplates one or more nucleic acid(s) encoding the peptides of the invention (e.g. S100A9, Insulin, and/or Affinity Tag), variants or fragments thereof.

The present invention also contemplates a gene delivery vector, preferably in the form of a plasmid or a vector, which comprises one or more nucleic acid(s) encoding the peptides of the invention (e.g. S100A9, Insulin, and/or Affinity Tag), variants or fragments thereof.

As used herein, a "vector" is capable of transferring nucleic acid sequences to target cells (e.g., viral vectors, non-viral vectors, particulate carriers, and liposomes).

Suitable vectors include derivatives of SV40 and known bacterial plasmids, e. g., E. coli plasmids col El, pCRl, pBR322, pLive, pMB9 and their derivatives, plasmids such as RP4; phage DNAs, e. g., the numerous derivatives of phage X, e. g., NM989, and other phage DNA, e. g., Ml 3 and filamentous single stranded phage DNA; yeast plasmids such as the 2µ plasmid or derivatives thereof; vectors useful in eukaryotic cells, such as vectors useful in insect or mammalian cells; vectors derived from combinations of plasmids and phage DNAs, such as plasmids that have been modified to employ phage DNA or other expression control sequences; and the like. Various viral vectors are used for delivering nucleic acid to cells in vitro or in vivo. Non-limiting examples are vectors based on Herpes Viruses, Pox- viruses, Adeno-associated virus, Lentivirus, and others. In principle, all of them are suited to deliver the expression cassette comprising an expressible nucleic acid molecule that codes for one or more nucleic acid(s) encoding the peptides (e.g. S100A9, Insulin, and/or Affinity Tag), variants or fragments thereof, of the invention.

Also contemplated in the present invention is a host cell comprising a plasmid or vector of the invention or one or more nucleic acid(s) encoding the peptides of the invention (e.g. S100A9, Insulin, and/or Affinity Tag), variants or fragments thereof. The host cell can be any prokaryotic or eukaryotic cell, preferably the host cell is a eukaryotic cell, most preferably the host cell is a mammalian cell. Even more preferably, the host cell is a human pancreatic cell.

The invention further provides pharmaceutical compositions comprising a therapeutically effective amount of a composition of the invention, or ii) a plasmid or a vector of the invention, or iii) a host cell of the invention, and a pharmaceutically acceptable excipient, diluent, carrier, salt and/or additive.

Usually, the pharmaceutical composition of the invention is for use in the treatment of an insulin deficiency (ID) condition, or an associated symptom, in a subject in need thereof.

The term " therapeutically effective amount" as used herein means an amount of a composition of the invention high enough to significantly positively modify the symptoms and/or condition to be treated, but low enough to avoid serious side effects (at a reasonable risk/benefit ratio), within the scope of sound medical judgment.

The therapeutically effective amount of the composition of the invention is selected in accordance with a variety of factors including type, species, age, weight, sex and medical condition of the patient; the severity of the condition to be treated; the route of administration; the renal and hepatic function of the patient. A physician of ordinary skill in the art can readily determine and prescribe the effective amount of the drug required to prevent, counter or arrest the progress of an insulin deficiency (ID) condition, or an associated symptom.

"Pharmaceutically acceptable carrier or diluent" means a carrier or diluent that is useful in preparing a pharmaceutical composition that is generally safe, non-toxic, and desirable, and includes carriers or diluents that are acceptable for human pharmaceutical use.

Such pharmaceutically acceptable carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions.

Pharmaceutically acceptable excipients include starch, glucose, lactose, sucrose, sodium stearate, glycerol monostearate, talc, sodium chloride, dried skim milk, glycerol, propylene glycol, water, ethanol, phenol, protamine sulphate, zinc oxide and the like.

The pharmaceutical compositions may further contain one or more pharmaceutically acceptable salts such as, for example, a mineral acid salt such as a hydrochloride, a hydrobromide, a phosphate, a sulfate, etc.; and the salts of organic acids such as acetates, propionates, malonates, benzoates, etc. Additionally, auxiliary substances, such as wetting or emulsifying agents, pH buffering substances, gels or gelling materials, flavorings, colorants, microspheres, polymers, suspension agents, etc. may also be present herein. In addition, one or more other conventional pharmaceutical ingredients, such as preservatives, humectants, suspending agents, surfactants, antioxidants, anticaking agents, fillers, chelating agents, coating agents, chemical stabilizers, etc. may also be present, especially if the dosage form is a reconstitutable form. Suitable exemplary ingredients include macrocrystalline cellulose, carboxymethyf cellulose sodium, polysorbate 80, phenyletbyl alcohol, chiorobutanol, potassium sorbate, sorbic acid, sulfur dioxide, propyl gallate, the parabens, ethyl vanillin, glycerin, phenol, parachlorophenol, gelatin, albumin and a combination thereof. A thorough discussion of pharmaceutically acceptable excipients is available in REMINGTON'S PHARMACEUTICAL SCIENCES (Mack Pub. Co., N.J. 1991) which is incorporated by reference herein.

The invention also provides the use of compositions and pharmaceutical compositions of the invention in the preparation of a medicament for the treatment of an insulin deficiency (ID) condition, or an associated symptom.

The compositions or pharmaceutical compositions of the invention, are administered concomitantly, separately or staggered.

Combined or concomitant administration can include co-administration, either in a single pharmaceutical formulation or using separate formulations, or consecutive administration in either order but generally within a time period such that all active agents can exert their biological activities simultaneously. Preparation and dosing schedules for such agents can be used according to manufacturers' instructions or as determined empirically by the skilled practitioner.

The present invention further provides a method of treating an insulin deficiency (ID) condition, or an associated symptom, in a subject in need thereof, the method comprising administering to the subject a therapeutically-effective amount of
i) a S100 calcium-binding protein A9 (S100A9), a variant or a fragment thereof and
ii) insulin, a variant or a fragment thereof.

In certain aspects, the treatment comprises increasing hepatic modified S100A9 mRNA level, increasing hepatic modified S100A9 protein level, increasing plasmatic modified S100A9 protein level, alleviating glucagonemia, alleviating ketonemia, alleviating triglyceridemia, decreasing circulating non-esterified fatty acids (NEFAs) level, alleviating hyperketonemia, alleviating hepatic fatty acid oxidation (FAO), increasing hepatic ATP level, decreasing hepatic mitochondrial DNA level, increasing lifespan, decreasing calprotectin level, alleviating hyperglycemia, alleviating hypertriglyceridemia, alleviating hyperglucagonemia, alleviating hypercalprotectinemia, alleviating hypoleptinemia, reducing body fat mass, alleviating hyperphagia, alleviating polydipsia, or any combination thereof.

In certain aspects, the treatment comprises decreasing the insulin dose by at least 5%, by at least 10%, by at least 15%, by at least 20%, by at least 25%, by at least 30%, by at least 35%, by at least 40%, by at least 45%, by at least 50%, or more as compared to the administration of insulin in the absence of a S100A9 protein, a variant or a fragment thereof.

The aforementioned reduced insulin doses in combination of S100A9 protein, a variant or a fragment thereof is able to achieve similar or better metabolic control as compared to 100% insulin dose in the absence of a S100A9 protein, a variant or a fragment thereof.

**Table 1**

| **SEQ ID NO:** | **Name** | **Sequence** |
|---|---|---|
| 1 | S100A9 | MTCKMSQLERNIETIINTFHQYSVKLGHPDTLNQGEFKELVRKDLQN FLKKENKNEKVIEHIMEDLDTNADKQLSFEEFIMLMARLTWASHEK MHEGDEGPGHHHKPGLGEGTP |
| 2 | S100A9 N91 | MTCKMSQLERNIETIINTFHQYSVKLGHPDTLNQGEFKELVRKDLQN FLKKENKNEKVIEHIMEDLDTNADKQLSFEEFIMLMARLTWASH |
| 3 | S100A9 C91 | MKLGHPDTLNQGEFKELVRKDLQNFLKKENKNEKVIEHIMEDLDTN ADKQLSFEEFIMLMARLTWASHEKMHEGDEGPGHHHKPGLGEGTP |
| 4 | S100A9 N76 | MTCKMSQLERNIETIINTFHQYSVKLGHPDTLNQGEFKELVRKDLQN FLKKENKNEKVIEHIMEDLDTNADKQLSF |
| 5 | S100A9 C76 | MLVRKDLQNFLKKENKNEKVIEHIMEDLDTNADKQLSFEEFIMLMA RLTWASHEKMHEGDEGPGHHHKPGLGEGTP |
| 6 | S100A9 N69A E78A | MTCKMSQLERNIETIINTFHQYSVKLGHPDTLNQGEFKELVRKDLQN FLKKENKNEKVIEHIMEDLDTAADKQLSFEAFIMLMARLTWASHEK MHEGDEGPGHHHKPGLGEGTP |
| 7 | Insulin Human A chain | GIVEQCCTSICSLYQLENYCN |
| 8 | Insulin Human B chain | FVNQHLCGSHLVEALYLVCGERGFFYTPKT |
| 9 | Insulin Lispro A chain | GIVEQCCTSICSLYQLENYCN |
| 10 | Insulin Lispro B chain | FVNQHLCGSHLVEALYLVCGERGFFYTKPT |
| 11 | Insulin Glulisine A chain | GIVEQCCTSICSLYQLENYCN |
| 12 | Insulin Glulisine B chain | FVKQHLCGSHLVEALYLVCGERGFFYTPET |
| 13 | Insulin Aspart A chain | GIVEQCCTSICSLYQLENYCN |
| 14 | Insulin Aspart B chain | FVNQHLCGSHLVEALYLVCGERGFFYTDKT |
| 15 | Insulin Glargine A chain | GIVEQCCTSICSLYQLENYCG |
| 16 | Insulin Glargine B chain | FVNQHLCGSHLVEALYLVCGERGFFYTPKTRR |
| 17 | Insulin Detemir A chain | GIVEQCCTSICSLYQLENYCN |
| 18 | Insulin Detemir B chain | FVNQHLCGSHLVEALYLVCGERGFFYTPK |
| | | myristic acid [CH3(CH2)12COOH] is added to the epsilon-amino group of K in position 29 of chain B |
| 19 | Insulin Degludec A chain | GIVEQCCTSICSLYQLENYCN |
| 20 | Insulin Degludec B chain | FVNQHLCGSHLVEALYLVCGERGFFYTPK |
| | | 16-carbon fatty acid is added to the epsilon-amino group of K in position 29 of chain B via a gamma-glutamic acid linker |
| 21 | 1xFLAG | DYKDDDDK |
| 22 | 2xFLAG | DYKDHD-G-DYKDHD |
| 23 | 3xFLAG | DYKDHD-G-DYKDHD-I-DYKDDDDK |
| 24 | CBP | TNPGVSAWQVNTAYTAGQLVTYNGKTYKCLQPHTSLAGWEPSNVP ALWQLQ |
| 25 | MBP | MKIEEGKLVIWINGDKGYNGLAEVGKKFEKDTGIKVTVEHPDKLEE KFPQVAATGDGPDIIFWAHDRFGGYAQSGLLAEITPDKAFQDKLYPF TWDAVRYNGKLIAYPIAVEALSLIYNKDLLPNPPKTWEEIPALDKEL KAKGKSALMFNLQEPYFTWPLIAADGGYAFKYENGKYDIKDVGVD NAGAKAGLTFLVDLIKNKHMNADTDYSIAEAAFNKGETAMTINGP WAWSNIDTSKVNYGVTVLPTFKGQPSKPFVGVLSAGINAASPNKEL AKEFLENYLLTDEGLEAVNKDKPLGAVALKSYEEELAKDPRIAATM ENAQKGEIMPNIPQMSAFWYAVRTAVINAASGRQTVDEALKDAQTN SSSNNNNNNNNNNLGIEGR |
| 26 | C-myc | EQKLISEEDL |
| 27 | SBP | MDEKTTGWRGGHWEGLAGELEQLRARLEHHPQGQREP |
| 28 | S | KETAAAKFERQHMDS |
| 29 | HAT | KDHLIHNVHKEFHAHAHNK |
| 30 | Calmodulin binding peptide | KRRWKKNFIAVSAANRFKKISSSGAL |
| 31 | Strep-tag II | WSHPQFEK |
| 32 | GST | MSPILGYWKIKGLVQPTRLLLEYLEEKYEEHLYERDEGDK WRNKKFELGLEFPNLPYYIDGDVKLTQSMAIIRYIADKHN MLGGCPKERAEISMLEGAVLDIRYGVSRIAYSKDFETLKV DFLSKLPEMLKMFEDRLCHKTYLNGDHVTHPDFMLYDALD VVLYMDPMCLDAFPKLVCFKKRIEAIPQIDKYLKSSKYIA WPLQGWQATFGGGDHPPKSDLVPRGSPGIHRD |
| 33 | Poly-His | HHHHHH |
| 34 | Full S100A9 + FLAG | MTCKMSQLERNIETIINTFHQYSVKLGHPDTLNQGEFKELVRKDLQN FLKKENKNEKVIEHIMEDLDTNADKQLSFEEFIMLMARLTWASHEK MHEGDEGPGHHHKPGLGEGTPDYKDDDDK |
| 35 | FLAG + Full S100A9 | MDYKDDDDKMTCKMSQLERNIETIINTFHQYSVKLGHPDTLNQGEF KELVRKDLQNFLKKENKNEKVIEHIMEDLDTNADKQLSFEEFIMLM ARLTWASHEKMHEGDEGPGHHHKPGLGEGTP |
| 36 | S100A9 N91 Fragment + FLAG | MTCKMSQLERNIETIINTFHQYSVKLGHPDTLNQGEFKELVRKDLQN FLKKENKNEKVIEHIMEDLDTNADKQLSFEEFIMLMARLTWASHDY KDDDDK |
| 37 | FLAG + S100A9 N91 Fragment | MDYKDDDDKMTCKMSQLERNIETIINTFHQYSVKLGHPDTLNQGEF KELVRKDLQNFLKKENKNEKVIEHIMEDLDTNADKQLSFEEFIMLM ARLTWASH |
| 38 | S100A9 N76 Fragment + FLAG | MTCKMSQLERNIETIINTFHQYSVKLGHPDTLNQGEFKELVRKDLQN FLKKENKNEKVIEHIMEDLDTNADKQLSFDYKDDDDK |
| 39 | FLAG + S100A9 N76 Fragment | MDYKDDDDKMTCKMSQLERNIETIINTFHQYSVKLGHPDTLNQGEF KELVRKDLQNFLKKENKNEKVIEHIMEDLDTNADKQLSF |
| 40 | FLAG + S100A9 C91 Fragment | MDYKDDDDKMKLGHPDTLNQGEFKELVRKDLQNFLKKENKNEKVI EHIMEDLDTNADKQLSFEEFIMLMARLTWASHEKMHEGDEGPGHH HKPGLGEGTP |
| 41 | S100A9 C91 Fragment + FLAG | MKLGHPDTLNQGEFKELVRKDLQNFLKKENKNEKVIEHIMEDLDTN ADKQLSFEEFIMLMARLTWASHEKMHEGDEGPGHHHKPGLGEGTP DYKDDDDK |
| 42 | S100A9 C76 Fragment + FLAG | MLVRKDLQNFLKKENKNEKVIEHIMEDLDTNADKQLSFEEFIMLMA RLTWASHEKMHEGDEGPGHHHKPGLGEGTPDYKDDDDK |
| 43 | FLAG + S100A9 C76 Fragment | MDYKDDDDKMLVRKDLQNFLKKENKNEKVIEHIMEDLDTNADKQL SFEEFIMLMARLTWASHEKMHEGDEGPGHHHKPGLGEGTP |
| 44 | S100A9 N69A E78A + FLAG | MTCKMSQLERNIETIINTFHQYSVKLGHPDTLNQGEFKELVRKDLQN FLKKENKNEKVIEHIMEDLDTAADKQLSFEAFIMLMARLTWASHEK MHEGDEGPGHHHKPGLGEGTPDYKDDDDK |
| 45 | Seq. cloned into pLIVE between BamH1 and Xho1 sites | GCTAGCGGATCCGCCGCCACCATGGCCAACAAAGCACCTTCTCAG ATGGAGCGCAGCATAACCACCATCATCGACACCTTCCATCAATAC TCTAGGAAGGAAGGACACCCTGACACCCTGAGCAAGAAGGAATT CAGACAAATGGTGGAAGCACAGTTGGCAACCTTTATGAAGAAAG AGAAGAGAAATGAAGCCCTCATAAATGACATCATGGAGGACCTG GACACAAACCAGGACAATCAGCTGAGCTTTGAGGAGTGTATGAT GCTGATGGCAAAGTTGATCTTTGCCTGTCATGAGAAGCTGCATGA GAACAACCCACGTGGGCATGGCCACAGTCATGGCAAAGGCTGTG GGAAGGACTACAAAGACGATGACGACAAGTGACTCGAG |
| 46 | S100A9 C3S P114C | MTSKMSQLERNIETIINTFHQYSVKLGHPDTLNQGEFKELVRKDLQN FLKKENKNEKVIEHIMEDLDTNADKQLSFEEFIMLMARLTWASHEK MHEGDEGPGHHHKPGLGEGTC |
| 47 | Peptidyl linker | GSSGSSGSSGSSGSSG |
| 48 | Avi-Tag | GLNDIFEAQKIEWHE |

While certain features of this invention have been illustrated and described herein, many modifications, substitutions, changes, and equivalents will now occur to those of ordinary skill in the art. It is, therefore, to be understood that the appended claims are intended to cover all such modifications and changes as fall within the true spirit of this invention.

### REFERENCES

1. Wasserfall, C., et al. Persistence of Pancreatic Insulin mRNA Expression and Proinsulin Protein in Type 1 Diabetes Pancreata. Cell metabolism 26, 568-575 e563 (2017).
2. Harris, K., Boland, C., Meade, L. & Battise, D. Adjunctive therapy for glucose control in patients with type 1 diabetes. Diabetes Metab Syndr Obes 11, 159-173 (2018).
3. Lyons, S.K., et al. Use of Adjuvant Pharmacotherapy in Type 1 Diabetes: International Comparison of 49,996 Individuals in the Prospective Diabetes Follow-up and T1D Exchange Registries. Diabetes Care 40, e139-e140 (2017).
4. Coppari, R. & Bjorbaek, C. Leptin revisited: its mechanism of action and potential for treating diabetes. Nature reviews. Drug discovery 11, 692-708 (2012).
5. Larsen, J., et al. Silent coronary atheromatosis in type 1 diabetic patients and its relation to long-term glycemic control. Diabetes 51, 2637-2641 (2002).
6. Orchard, T.J., et al. Insulin resistance-related factors, but not glycemia, predict coronary artery disease in type 1 diabetes: 10-year follow-up data from the Pittsburgh Epidemiology of Diabetes Complications Study. Diabetes care 26, 1374-1379 (2003).
7. Umpierrez, G. & Korytkowski, M. Diabetic emergencies - ketoacidosis, hyperglycaemic hyperosmolar state and hypoglycaemia. Nature reviews. Endocrinology 12, 222-232 (2016).
8. Banting, F.G., Best, C.H., Collip, J.B., Campbell, W.R. & Fletcher, A.A. Pancreatic Extracts in the Treatment of Diabetes Mellitus. Canadian Medical Association journal 12, 141-146 (1922).
9. Banting, F.G., Campbell, W.R. & Fletcher, A.A. Further Clinical Experience with Insulin (Pancreatic Extracts) in the Treatment of Diabetes Mellitus. British medical journal 1, 8-12 (1923).
10. Horton, J.D., Goldstein, J.L. & Brown, M.S. SREBPs: activators of the complete program of cholesterol and fatty acid synthesis in the liver. The Journal of clinical investigation 109, 1125-1131 (2002).
11. Shulman, G.I. Cellular mechanisms of insulin resistance. The Journal of clinical investigation 106, 171-176 (2000).
12. Bulsara, M.K., Holman, C.D., Davis, E.A. & Jones, T.W. The impact of a decade of changing treatment on rates of severe hypoglycemia in a population-based cohort of children with type 1 diabetes. Diabetes Care 27, 2293-2298 (2004).
13. Cryer, P.E. Hypoglycemia-associated autonomic failure in diabetes. American journal of physiology. Endocrinology and metabolism 281, E1115-1121 (2001).
14. Cryer, P.E. Mechanisms of hypoglycemia-associated autonomic failure and its component syndromes in diabetes. Diabetes 54, 3592-3601 (2005).
15. Chiang, J.L., Kirkman, M.S., Laffel, L.M., Peters, A.L. & Type 1 Diabetes Sourcebook, A. Type 1 diabetes through the life span: a position statement of the American Diabetes Association. Diabetes Care 37, 2034-2054 (2014).
16. Averill, M.M., Kerkhoff, C. & Bornfeldt, K.E. S100A8 and S100A9 in cardiovascular biology and disease. Arteriosclerosis, thrombosis, and vascular biology 32, 223-229 (2012).
17. Loser, K., et al. The Toll-like receptor 4 ligands Mrp8 and Mrp14 are crucial in the development of autoreactive CD8+ T cells. Nature medicine 16, 713-717 (2010).
18. Leanderson, T., Liberg, D. & Ivars, F. S100A9 as a Pharmacological Target Molecule in Inflammation and Cancer. Endocrine, metabolic & immune disorders drug targets 15, 97-104 (2015).
19. Eggers, K., et al. RAGE-dependent regulation of calcium-binding proteins S100A8 and S100A9 in human THP-1. Experimental and clinical endocrinology & diabetes : official journal, German Society of Endocrinology [and] German Diabetes Association 119, 353-357 (2011).
20. Geczy, C.L., Chung, Y.M. & Hiroshima, Y. Calgranulins may contribute vascular protection in atherogenesis. Circulation journal: official journal of the Japanese Circulation Society 78, 271-280 (2014).
21. Vogl, T., et al. Mrp8 and Mrp14 are endogenous activators of Toll-like receptor 4, promoting lethal, endotoxin-induced shock. Nature medicine 13, 1042-1049 (2007).
22. Vogl, T., et al. Autoinhibitory regulation of S100A8/S100A9 alarmin activity locally restricts sterile inflammation. J Clin Invest 128, 1852-1866 (2018).
23. Blanco-Rojo, R., et al. Interaction of an S100A9 gene variant with saturated fat and carbohydrates to modulate insulin resistance in 3 populations of different ancestries. The American journal of clinical nutrition 104, 508-517 (2016).
24. Ortega, F.J., et al. Targeting the association of calgranulin B (S100A9) with insulin resistance and type 2 diabetes. J Mol Med (Berl) 91, 523-534 (2013).
25. Nyalwidhe, J.O., et al. Comparative quantitative proteomic analysis of disease stratified laser captured microdissected human islets identifies proteins and pathways potentially related to type 1 diabetes. PloS one 12, e0183908 (2017).
26. Zhi, W., et al. Discovery and validation of serum protein changes in type 1 diabetes patients using high throughput two dimensional liquid chromatography-mass spectrometry and immunoassays. Molecular & cellular proteomics : MCP 10, M111 012203 (2011).
27. Fujikawa, T., et al. Leptin engages a hypothalamic neurocircuitry to permit survival in the absence of insulin. Cell metabolism 18, 431-444 (2013).

### EXAMPLES

### Material & Methods

**Animals and induction of insulin-deficiency.** All mice were maintained with standard chow diet and water available *ad libitum* in a light- and temperature-controlled environment. All the experiments described in the study used adult male mice. Insulin deficient animal models were generated as follows: Diphtheria Toxin (DT, Sigma Aldrich) was dissolved in sterile 0.9% NaCl and intraperitoneally administrated into *RIP-DTR* animals (0.5 ug/kg body weight at days 0, 1, 4).
**Assessment of mRNA, protein, and substrates content.** Mice were sacrificed and their tissues quickly removed and snap-frozen in liquid nitrogen and subsequently stored at -80°C. RNAs were extracted using Trizol reagent (Invitrogen). Complementary DNA was generated by Superscript II (Invitrogen) and used with SYBR Green PCR master mix (Applied Biosystem, Foster City, CA, USA) for quantitative real time PCR (q-RTPCR) analysis. mRNA contents were normalized to *18s* mRNA levels. All assays were performed using an Applied Biosystems QuantStudio® 5 Real-Time PCR System. For each mRNA assessment, q-RTPCR analyses were repeated at least 3 times. Proteins were extracted by homogenizing samples in lysis buffer (Tris 20mM, EDTA 5mM, NP40 1% (v/v), protease inhibitors (P2714-1BTL from Sigma, St. Louis, MO, USA), then resolved by SDS-PAGE and finally transferred to a nitrocellulose membrane by electroblotting. The following antibodies were used: CalgranulinB-S100A9 (cat. Number PB9678, Boster).
**Circulating substrates and hormones levels.** Tail vein blood was collected between 2 and 4 PM from mice that were fed *ad libitum.* To avoid random post-prandial confounding effects food was removed 2 hours prior to blood collection. Serum or plasma samples were collected after centrifugation (3500xg, 10 min) and stored at -80°C. Glucose, non-esterified fatty acids, triglycerides, ketone bodies, and glucagon levels were measured using commercially available kits.
**Overexpression of S100A9.** Hydrodynamic tail vein injection (HTVI) was performed. Overexpression of S100A9 was achieved by using pLIVE vectors (Myrus) that allow expression of a given gene under the control of the albumin promoter. The following sequences were cloned into pLIVE between restriction sites BamH1 and Xho1: 
GCTAGCGGATCCGCCGCCACCATGGCCAACAAAGCACCTTCTCAGATGGAGCGCAGCAT
AACCACCATCATCGACACCTTCCATCAATACTCTAGGAAGGAAGGACACCCTGACACCC
TGAGCAAGAAGGAATTCAGACAAATGGTGGAAGCACAGTTGGCAACCTTTATGAAGAA
AGAGAAGAGAAATGAAGCCCTCATAAATGACATCATGGAGGACCTGGACACAAACCAG
GACAATCAGCTGAGCTTTGAGGAGTGTATGATGCTGATGGCAAAGTTGATCTTTGCCTG
TCATGAGAAGCTGCATGAGAACAACCCACGTGGGCATGGCCACAGTCATGGCAAAGGC
TGTGGGAAGGACTACAAAGACGATGACGACAAGTGACTCGAG (SEQ ID No. 45).
 pLIVE-S100A9 plasmid DNA was sequenced to confirm correct sequences and orientation. Each mouse received 50 µg of pLIVE-S100A9 or pLIVE; aged-matched mice that did not undergo any procedure were used as healthy controls. Data shown in Fig. 2 were collected from mice injected intraperitoneally with 20 micro-grams of recombinant S100A9.
**Statistical analysis.** Data sets were analyzed for statistical significance using PRISM (GraphPad, San Diego, CA) for a two-tail unpaired Student's t test when two groups were compared or one-or two-way ANOVA (Tukey's post test) when more than two groups were compared.

### Results

### Beneficial metabolic actions of enhanced S100A9 in T1D mouse models

The Inventors overexpressed S100A9 in mice with insulin deficiency. The Inventors performed Hydrodynamic Tail Vein Injection studies in RIP-DTR mice that bear a rat insulin promoter (RIP) upstream of diphtheria toxin receptor (DTR) sequences cloned into the Hprt locus of the X chromosome. Following three consecutive intraperitoneal DT administrations, RIP-DTR mice develop a near-total-loss of pancreatic β-cells27. Indeed, almost all pancreatic β-cells were ablated in DT-injected RIP-DTR mice that underwent HTVI of either pLIVE (DT-pLIVE) or pLIVE-S100A9 (DT-pLIVE-S100A9) (data not shown). In line with β-cell loss, pancreatic Proinsulin mRNA level was barely measureable and these defects resulted in almost undetectable circulating insulin in DT-pLIVE and DT-pLIVE-S100A9 mice (Fig. 1a,b). To test whether DT-pLIVE-S100A9 mice have increased S100A9 we assessed plasmatic S100A9 level and found it to be increased in DT-pLIVE-S100A9 mice compared to DT-pLIVE and healthy controls (Fig. 1c). Collectively, these data demonstrate that DT-pLIVE-S100A9 mice are insulin deficient and overexpress S100A9. Owing to their ID, DT-pLIVE mice developed hyperglycemia, hyperketonemia, hypertriglyceridemia, and hyperglucagonemia (Figs. 1d-f). Next, the Inventors assessed the consequence of S100A9 overexpression on the aforementioned defects. Hyperglycemia was slightly improved in DT-pLIVE-S100A9 compared to DT-pLIVE mice (Fig. 1c). Remarkably, the circulating levels of glucagon, β-hydroxybutyrate, and triglycerides were all similar and significantly reduced between DT-pLIVE-S100A9 mice and healthy and DT-pLIVE controls, respectively (Fig. 1e-f).

### The therapeutic value of combination therapy between S100A9 and insulin in T1D

Results shown in Fig. 2 indicate a metabolic-improving and pro-survival action of enhanced S100A9 in T1D mice. The Inventors started testing whether enhanced S100A9 is able to reduce the insulin dose for management of T1D. Specifically, the Inventors increased circulating S100A9 level in combination with sub-optimal insulin dose (this is an insulin regimen that is not able to improve hyperglycemia and hyperketonemia caused by β-cell loss).

The results shown in Fig. 2 further indicate that while the sub-optimal dose of insulin did not affect hyperglycemia in control mice it significantly reduced hyperglycemia in mice overexpressing S100A9 without causing hypoglycemia.

## Claims

1. A composition for use in the treatment of insulin deficiency (ID) condition, or an associated symptom, in a subject in need thereof, the composition comprising
i) a S100 calcium-binding protein A9 (S100A9), a variant or a fragment thereof and
ii) insulin, a variant or a fragment thereof.

2. The composition for use of claim 1, wherein the S100A9 protein, variant or fragment thereof, comprises at least one affinity tag.

3. The composition for use of claim 2, wherein the at least one affinity tag is attached to the C' and/or the N' terminus of the S100A9 protein, variant or fragment thereof.

4. The composition for use of claim 2 or 3, wherein the affinity FLAG tag (SEQ ID NO: 21), chitin binding protein (CBP) tag (SEQ ID NO: 24), maltose binding protein (MBP) tag (SEQ ID NO: 25), Strep tag II (SEQ ID NO: 31), glutathione-S-transferase (GST) tag (SEQ ID NO: 32), poly(His) tag (SEQ ID NO: 33), C-myc (SEQ ID NO: 26), SBP (SEQ ID NO: 27), S (SEQ ID NO: 28), HAT (SEQ ID NO: 29), and a combination of one more thereof..

5. The composition for use of any one of the preceding claims, wherein i) the S100A9 protein, variant or fragment thereof, ii) insulin, variant or fragment thereof, and iii) at least one affinity tag are present on the same peptide.

6. The composition for use of any one of the preceding claims, wherein the amino-acid sequence of the S100A9 protein is as set forth in SEQ ID NO: 1.

7. The composition for use of any one of the preceding claims, wherein the amino-acid sequence of the S100A9 protein variant differs from the amino-acid sequence set forth in SEQ ID NO: 1, or from an active fragment thereof, in 1 to 10 amino acids.

8. The composition for use of any one of the preceding claims, wherein the fragment of the S100A9 protein is an active fragment comprising at least 25 consecutive amino-acids, at least 30 consecutive amino-acids, at least 35 consecutive amino-acids, at least 40 consecutive amino-acids, at least 50 consecutive amino-acids, at least 60 consecutive amino-acids, at least 67 consecutive amino-acids, at least 70 consecutive amino-acids, at least 75 consecutive amino-acids, at least 80 consecutive amino-acids, at least 85 consecutive amino-acids, at least 91 consecutive amino-acids, at least 95 consecutive amino-acids, at least 100 consecutive amino-acids, at least 105 consecutive amino-acids, at least 110 consecutive amino-acids, at least 115 consecutive amino-acids, at least 120 consecutive amino-acids, at least 125 consecutive amino-acids or at least 130 consecutive amino-acids of the amino-acid sequence set forth in SEQ ID NO: 1.

9. The composition for use of any one of the preceding claims, wherein insulin is native insulin, proinsulin, basal insulin or bolus insulin.

10. The composition for use of any one of the preceding claims, wherein the amino-acid sequence of the insulin protein is as set forth in SEQ ID NO: 7 and/or SEQ ID NO: 8.

11. The composition for use of any one of the preceding claims, wherein the amino-acid sequence of the insulin protein variant differs from the amino-acid sequence set forth in SEQ ID NO: 7 and/or SEQ ID NO: 8, or from an active fragment thereof, in 1 to 10 amino acids.

12. The composition for use of any one of the preceding claims, wherein insulin deficiency associated symptom is selected from the group comprising hyperglycemia, hyperketonemia, ketoacidosis, hypertriglyceridemia, hyperglucagonemia, hypercalprotectinemia, increased or high circulating (non-esterified fatty acids (NEFAs) level, severe hypoleptinemia, reduced or low body fat mass, hyperphagia, polydipsia and any combination thereof.

13. The composition for use of any one of the preceding claims, wherein the insulin deficiency (ID) condition is diabetes 1 or diabetes 2.

14. The composition for use of any one of the preceding claims, wherein the treatment comprises alleviating hyperglycemia, alleviating and/or reducing risk of hypoglycemia, alleviating increased level of glycated hemoglobin in the blood, alleviating hyperglucagonemia, alleviating and/or reducing risk of hyperketonemia and ketoacidosis, alleviating hypertriglyceridemia, alleviating increased hepatic fatty acid oxidation (FAO), increasing hepatic native or modified S100A9 mRNA level, increasing hepatic native or modified S100A9 protein level, increasing plasmatic native or modified S100A9 protein level, increasing hepatic ATP level, increasing lifespan, decreasing circulating non-esterified fatty acids (NEFAs) level, decreasing hepatic mitochondrial DNA level, decreasing circulating calprotectin level, decreasing lipase activity, or any combination thereof.

15. The composition for use of any one of the preceding claims, wherein the i) S100A9 protein, variant or fragment thereof, and ii) the insulin, variant or fragment thereof, are administered concomitantly, separately or staggered.

16. The composition for use of any one of the preceding claims further comprising a sodium-glucose cotransporter 1 (SGLT1) and/or 2 (SGLT2) inhibitor(s), amylin analogs, biguanides (e.g., metformin), incretin mimetics (e.g., glucagon-like peptide receptor agonists, dipeptidyl-peptidase-4 inhibitors).
